# FASCICULE DE BREVET EUROPEEN

(11) **EP 1 529 523 B1**
(45) Date de publication et mention de la délivrance du brevet: **16.08.2006**
(21) Numéro de dépôt: 04292589.1
(22) Date de dépôt: 02.11.2004
(51) Int. Cl.: A61Q 17/00, A61Q 19/00, A61K 8/34, A61K 8/02, A61K 8/44

(54) **Utilisation cosmétique du phytantriol comme agent empêchant ou réduisant l'adhésion des microorganismes sur la surface de la peau et /ou des muqueuses**
Kosmetische Vewendung von Phytantriol als Mittel zur Verhinderung oder Verminderung des Anhafstens von Mikroorganismen auf der Oberfläche der Haut und/oder der Schleimhäute.
Cosmetic use of phytantriol as an agent for preventing or reducing the adhesion of microorganisms on the skin and/or mucosal surfaces

(30) Priorité: 07.11.2003 FR 0350809
(43) Date de publication de la demande: 11.05.2005
(73) Titulaire: L'ORÉAL, 75008 Paris (FR)
(72) Inventeur: Potin, Anthony, 75006 Paris (FR)
(74) Mandataire: Kromer, Christophe

(56) Documents cités:
- EP-A- 0 686 386
- EP-A- 0 711 540
- EP-A- 1 133 979
- EP-A- 1 161 938
- WO-A-00/62741
- WO-A-96/23479
- WO-A-99/62475

## Description

La présente demande se rapporte à l'utilisation du phytantriol dans une composition cosmétique ou pour la préparation d'une composition dermatologique en tant qu'agent empêchant ou réduisant l'adhésion des microorganismes sur la surface de la peau et/ou des muqueuses.

La peau humaine est peuplée en permanence d'une multitude de microorganismes différents (bactéries, levures et champignons). Les microorganismes commensaux vivant sur ou dans la peau peuvent faire partie d'une microflore soit résidente (normale), soit transitoire. La flore microbienne résidente, indispensable à la bonne santé de la peau, est constituée principalement de staphylocoques (*Staphylococcus epidermis* et *Staphylococcus hominis*), de corynebactéries, de propionibactéries Gram+ telles que *Propionibacterium acnes*, ainsi que d'une flore fongique principalement composée de *Pytosporum ovale.* Leur présence est établie en profils de distribution bien définis. Habituellement, les micro-organismes transitoires ne se fixent pas fermement; ils sont incapables de se multiplier et meurent normalement après quelques heures.

L'anatomie et la physiologie de la peau varient d'une partie à l'autre du corps et la microflore résidante reflète ces variations.

La plupart des bactéries de la peau sont présentes sur l'épiderme squameux superficiel, colonisant les cellules mortes et étroitement associées aux glandes sébacées et sudoripares. Les excrétions de ces glandes fournissent de l'eau, des acides aminés, de l'urée, des électrolytes et des acides gras spécifiques servant d'éléments nutritifs principalement pour *Staphylococcus epidermidis* et des corynébactéries aérobies.

Les infections cutanées sont le plus souvent dues à la rupture de l'équilibre écologique de la flore résidente suite à la colonisation de la peau par des germes exogènes pathogènes ou à la prolifération anormale d'une souche endogène. Les germes pathogènes les plus connus sont *Pseudomonas aeruginosa* (Gram -) qui est responsable de petits boutons, de folliculites, de rougeurs et de prurit, *Candida albicans* pouvant provoquer des inflammations à la commissure des lèvres, candidoses cutanées, du prurit, des folliculites et des aphtes, *Staphylococcus aureus* pouvant provoquer des boutons, des folliculites, de l'impétigo, et des furoncles, et *Streptococcus* du groupe A responsable d'impétigo.

Pour combattre ces micro-organismes, il est courant d'utiliser des antibiotiques ou des bactéricides. L'utilisation de ces composés pose cependant le problème de la non spécificité d'action visant indifféremment la flore pathogène et la flore résidente, le problème du risque d'apparition de résistances bactériennes, ainsi que des problèmes de tolérance cutanée (irritations, allergies).

Il est également connu de réduire ou de prévenir la colonisation de surfaces telles que les dents, la peau et/ou les muqueuses, par des germes pathogènes en empêchant leur fixation sur ces supports. Les composés utilisés en tant qu'agents anti-adhésion décrits dans l'art antérieur sont soit des silicones (WO-99/62475), soit des hydrates de carbone et des dérivés d'hydrates de carbone, tels que ceux décrits dans la demande de brevet WO-96/23479, soit des huiles végétales comme décrit dans la demande de brevet EP-1 133 979.

Or, la plupart des hydrates de carbone constituent une source de carbone pour des bactéries et champignons. Leur présence dans des compositions cosmétiques favorise par conséquent la prolifération microbienne et nécessite l'augmentation de la concentration en agents conservateurs (bactéricides ou bactériostatiques). Cet inconvénient annule ainsi le bénéfice de l'approche consistant à remplacer des composés antibiotiques ou bactéricides par des composés réduisant l'adhérence microbienne.

La demanderesse a mis en évidence que, de façon surprenante, le phytantriol permettait de réduire de manière significative l'adhésion des microorganismes à la surface de la peau et/ou des muqueuses, et de prévenir ainsi la prolifération de germes potentiellement pathogènes en l'absence d'agent antibiotiques, bactéricides ou fongicides, et sans qu'il soit nécessaire d'utiliser une quantité importante d'agents conservateurs.

Le phytantriol ou 3,7,11,15-tétraméthyl-1,2,3-hexadecanetriol est un composé connu, notamment commercialisé sous la dénomination de « Phytantriol-63926 » par la Société Roche. Il a été utilisé dans des compositions cosmétiques, notamment en association avec le farnesol et au moins un autre agent actif afin de réguler les discontinuités visibles ou tactiles de la peau (WO-2000/062745). Il a également été décrit dans la demande EP-A-1161938 comme agent capable de limiter la pénétration dans la peau des polluants, la protégeant ainsi contre les effets délétères de la pollution.

Le phytantriol, à la différence des hydrates de carbone qui se lient aux récepteurs des micro-organismes pour empêcher les liaisons aux glycolipides des cornéocytes, agit sur les propriétés physico-chimiques de la surface de la peau et/ou des muqueuses, ces propriétés physico-chimiques faisant intervenir les interactions électrodynamiques dues aux forces de Van der Waals, les interactions acido-basiques selon Lewis et les interactions électrostatiques.

L'invention a donc pour objet l'utilisation cosmétique non thérapeutique du phytantriol dans une composition en tant qu'agent empêchant ou réduisant l'adhésion des microorganismes sur la surface de la peau et/ou des muqueuses.

L'invention a encore pour objet l'utilisation du phytantriol pour la préparation d'une composition dermatologique destinée à prévenir ou lutter contre les pathologies liées à l'adhésion des microorganismes sur la surface de la peau et/ou des muqueuses.

Par "*empêcher ou réduire l'adhésion des micro-organismes*", il faut entendre que le phytantriol ou la composition le contenant peut être utilisé aussi bien à titre préventif, pour sa capacité à prévenir, totalement ou partiellement, l'adhésion des micro-organismes, qu'à titre curatif pour sa capacité à faciliter le détachement des micro-organismes.

Le phytantriol peut être présent dans la composition pour application topique, en une quantité allant par exemple de 0,001 à 20 % en poids et de préférence de 0,1 à 10 % en poids par rapport au poids total de la composition.

Le phytantriol utilisé selon l'invention peut être incorporé notamment dans des compositions pouvant se présenter en particulier sous forme d'émulsions huile-dans-eau (H/E) ou eau-dans-huile (E/H) ou sous forme de particules de gel cubique, ces dernières pouvant être utilisées seules ou incorporées dans une émulsion.

Le terme de gel cubique désigne des gels transparents, isotropes en lumière polarisée, se présentant sous forme de phase cristal liquide cubique. Les phases cubiques sont organisées d'une manière bi-polaire en domaines hydrophile et lipophile distincts, en contact étroit et formant un réseau tridimensionnel thermodynamiquement stable. Une telle organisation a été notamment décrite dans Luzzati (1968), « Biological Membranes » (Chapman, D. Ed.), vol 1, 71-123 et dans Mariani et coll. (1988), J. Mol. Biol., 204, 165-189, ainsi que dans "La Recherche" (1992), Vol.23, 306-315. Selon l'arrangement des domaines hydrophile et lipophile, la phase cubique est dite de type normal ou inverse. Le terme de gel cubique utilisé selon la présente invention regroupe bien entendu les gels présentant les différents types de phases cubiques.

Quand le gel cubique est dispersé dans un milieu aqueux, on obtient des particules de gel cubique en dispersion, particules qui ont la même structure que le gel cubique non dispersé.

Les particules de gel cubique contenant le phytantriol peuvent être présentes dans la composition pour application topique, en une quantité allant par exemple de 0,1 à 20 % en poids et de préférence de 0,1 à 10 % en poids par rapport au poids total de la composition.

De manière avantageuse, les particules de gel cubique contenant le phytantriol sont en dispersion aqueuse dans la composition. Elles peuvent être notamment obtenues selon le mode préféré de réalisation décrit ci-dessous.

Selon ce mode de réalisation, les particules se trouvent en dispersion aqueuse et sont formées par un mélange comprenant (i) de 0,1 à 15 % en poids par rapport au poids total de la composition, de 3,7,11,15-tétraméthyl-1,2,3-hexadecanetriol ou phytantriol, éventuellement associé à un dérivé N-2-alcoxycarbonyle de N-méthylglucamine et/ou à un monoglycéride d'acide gras insaturé, et (ii) de 0,05 à 3 % en poids par rapport au poids total de la composition, d'au moins un agent dispersant et stabilisant, ledit agent étant choisi parmi les agents tensioactifs hydrosolubles à température ambiante, contenant une chaîne grasse, saturée ou insaturée, linéaire ou ramifiée, ayant de 8 à 22 atomes de carbone. Les pourcentages sont exprimés ici par rapport à la quantité totale de la composition contenant les particules de gel cubique à base de phytantriol.

Selon ce mode de réalisation des particules de gel cubique utilisées selon l'invention, la proportion pondérale relative en phytantriol par rapport au poids de l'agent dispersant et stabilisant (ii) peut aller par exemple de 2 à 200, et est de préférence inférieure ou égale à 50 (notamment allant de 2 à 50).

Parmi les dérivés N-2-alcoxycarbonyles de N-méthylglucamine, on peut notamment citer ceux répondant à la formule (I) suivante : 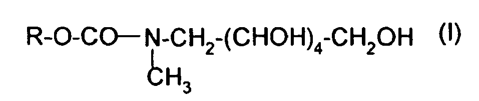 dans laquelle R représente un radical alkyle ramifié comportant de 6 à 18 atomes de carbone.

Parmi ceux-ci, on peut notamment mentionner la N-2-hexyldécyloxycarbonyl-N-méthyl-glucamine, la N-2-éthylhexyloxycarbonyl-N-méthylglucamine, la N-2-butyloctyloxycarbonyl-N-méthylglucamine et leurs mélanges.

Les composés de formule (I) tels que définis ci-dessus sont décrits et peuvent être préparés selon le procédé décrit dans le document EP-A-711540. Ce procédé comprend notamment les étapes consistant :
(a) à dissoudre dans un mélange d'eau et d'un solvant organique, de la N-méthylglucamine, le solvant pouvant être par exemple le tétrahydrofuranne,
(b) à disperser dans le mélange obtenu précédemment, du bicarbonate de sodium, en une proportion appropriée correspondant environ à quatre fois la proportion molaire de N-méthyl-glucamine,
(c) à introduire alors dans le mélange réactionnel obtenu, un chloroformiate d'alkyle, le radical alkyle étant en C6-C18, en une proportion appropriée, généralement équimolaire par rapport à celle de N-méthylglucamine, puis à laisser réagir le mélange, et
(d) à filtrer le mélange réactionnel obtenu après l'étape (c), à recueillir le résidu pâteux obtenu par filtration, puis à le dissoudre dans de l'acétone en vue de sa cristallisation à une température de l'ordre de 5°C. Après filtration, les cristaux du dérivé N-2-alcoxycarbonyle de N-méthylglucamine formé sont essorés et séchés sous vide.

Dans le cas où l'on utilise le phytantriol en mélange avec un ou des composés de formule (I), ce mélange comprend une quantité de phytantriol allant de 1 à 40 % en poids et mieux de 10 à 30 % en poids par rapport au poids du mélange, et une quantité de dérivé N-2-alcoxycarbonyle de N-méthylglucamine de formule (I) allant de 60 à 99 % en poids et mieux de 70 à 90 % en poids par rapport au poids du mélange.

Les monoglycérides d'acide gras insaturé pouvant être utilisés en mélange avec le phytantriol pour la préparation de particules de gel cubique sont de préférence ceux ayant une chaîne grasse insaturée comportant de 16 à 22 atomes de carbone. Parmi ceux-ci, on peut notamment citer le monooléate de glycéryle ou monooléine et le monolinoléate de glycéryle ou monolinoléine. On peut bien entendu utiliser pour préparer les dispersions de particules de gel cubique, un mélange de monoglycérides tels que définis précédemment ainsi qu'un mélange de monoglycérides d'acide gras insaturé et de monoglycérides d'acide gras saturé, la proportion en monoglycérides d'acide gras saturé étant cependant de préférence inférieure à celle de monoglycérides d'acide gras insaturé.

Dans le cas où l'on utilise du phytantriol en mélange avec des monoglycérides d'acide gras insaturé ce mélange comprend de préférence une quantité de phytantriol allant de 1 à 50 % en poids et mieux de 10 à 30 % en poids par rapport au poids total du mélange et une quantité de monoglycéride d'acide gras insaturé allant de 50 à 99 % en poids et mieux de 70 à 90 % en poids par rapport au poids du mélange.

L'agent dispersant et stabilisant (ii) des particules de gel cubique est de préférence choisi parmi :
(1) les alkyl ou alcényl éthers ou esters de polyol,
(2) les amino-acides N-acylés et leurs dérivés, et les peptides N-acylés par un radical alkyle ou alcényle et leurs sels,
(3) les alkyl ou alcényl éthers ou esters sulfates, leurs dérivés et leurs sels,
(4) les alkyl ou alcényl éthers ou esters gras polyoxyéthylénés,
(5) les acides alkyl ou alcényl carboxyliques polyoxyéthylénés et leurs sels,
(6) les N-alkyl ou alcényl bétaïnes,
(7) les alkyl ou alcényl triméthylammonium et leurs sels, et
(8) leurs mélanges.

Dans les composés ci-dessus énumérés, les radicaux alkyle et alcényle ont de préférence de 8 à 22 atomes de carbone et peuvent se présenter sous forme de mélanges.
(1) Comme alkyl ou alcényl éthers ou esters de polyol, on peut citer notamment :
   (a) les alkyl ou alcényl esters de sorbitan polyoxyéthylénés comportant au moins 20 motifs d'oxyde d'éthylène, tels que le palmitate de sorbitan 20 OE ou Polysorbate 40 commercialisé sous la dénomination de « Montanox 40 DF » par la Société Seppic, et le laurate de sorbitan 20 OE ou Polysorbate 20 commercialisé sous la dénomination de « Tween 20 » par la Société ICI,
   (b) les alkyl ou alcényl esters de polyglycérol comportant au moins 10 motifs dérivés du glycérol, oxyéthylénés ou non, tels que le polyglycéryl-10 laurate commercialisé sous la dénomination de « Decaglyn 1-L » par la Société Nikko Chemicals,
   (c) les alkyl ou alcényl éthers de polyglycérol, tels que le polyglycéryl-3 hydroxylauryléther commercialisé sous la dénomination de « Chimexane NF » par la Société Chimex, et
   (d) les alkyl ou alcényl éthers ou esters de mono ou polysaccharides, tels que ceux dérivant du glucose, du fructose, du galactose, du maltose ou du lactose et notamment les monoesters en 1- et 6- du D-fructose, du décylglucose et du décylpolyglucose.
(2) Comme amino-acides N-acylés et dérivés, et comme peptides N-acylés par un radical alkyle ou alcényle, et sels, on utilise de préférence ceux pour lesquels le radical alkyle ou alcényle comporte au moins 12 atomes de carbone.
   Par amino-acides, on entend selon l'invention les alpha-, bêta- ou gamma-amino-acides. Comme sels d'amino-acides N-acylés, on peut citer par exemple les sels de N-acylglutamate, tels que le cocoylglutamate de monosodium, le lauroylglutamate de monosodium, l'alcoyl C14-C20 glutamate de disodium (le radical alcoyle C14-C20 dérivant du suif hydrogéné), commercialisés respectivement sous les dénominations de « Acylglutamate CS-11 », de « Acylglutamate LS-11 » et de « Acylglutamate HS-21 » par la Société Ajinomoto. On peut encore citer les lysines N-acylées telles que la lauroyl-lysine commercialisée sous la dénomination de « Amihope LL » par la Société Ajinomoto. Les dérivés d'amino-acides N-acylés et leurs sels sont de préférence les sarcosinates N-acylés tels que le lauroyl-sarcosinate de sodium commercialisé sous la dénomination de « Oramix L30 » par la Société Seppic, le myristoylsarcosinate de sodium et le palmitoylsarcosinate de sodium commercialisés respectivement sous les dénominations de « Nikkol Sarcosinate MN » et de « Nikkol Sarcosinate PN » par la Société Nikko Chemicals.
   Parmi les peptides N-acylés, on peut citer ceux dérivés de tout ou partie du collagène ou de la kératine tels que le lauroyl collagène de sodium et la palmitoyl kératine commercialisés sous les dénominations de « Proteol B 30 » et de « Lipacide PK » par la Société Seppic.
(3) Parmi les alkyl ou alcényl éthers ou esters sulfates, leurs dérivés et leurs sels, on utilise de préférence ceux pour lesquels le radical alkyle ou alcényle comporte au moins 12 atomes de carbone.
   Parmi les alkyl ou alcényl éthers sulfates, on utilise de préférence les sels d'alkyl éther sulfate et notamment le lauryléther sulfate de sodium. Parmi les alkyl ou alcényl esters sulfates, on peut citer par exemple les esters de l'acide iséthionique ainsi que ses sels et notamment le cocoyl iséthionate de sodium commercialisé sous la dénomination de « Geropon AC 78 » par la Société Rhône Poulenc.
(4) Parmi les alkyl ou alcényl éthers ou esters gras polyoxyéthylénés, on utilise de préférence ceux pour lesquels le radical alkyle ou alcényle comporte au moins 12 atomes de carbone. Ceux particulièrement préférés ont au moins 20 motifs d'oxyde d'éthylène tels que par exemple le PEG-20 stéarate, le laureth-23, l'oleth-20 et le PEG-25 phytostérol.
(5) Parmi les acides alkyl ou alcényl carboxyliques polyoxyéthylénés et leurs sels, on utilise de préférence ceux comportant au moins 10 motifs d'oxyde d'éthylène tels que par exemple l'acide laureth-10 carboxylique et l'acide oleth-10 carboxylique.
(6) Parmi les N-alkyl ou alcényl bétaïnes, on utilise de préférence celles pour lesquelles le radical alkyle ou alcényle comporte au moins 12 atomes de carbone telles que par exemple la lauryl-amidopropyl bétaïne et l'oléyl-amidopropyl bétaïne.
(7) Parmi les alkyl ou alcényl triméthylammonium et leurs sels, on utilise de préférence ceux pour lesquels le radical alkyle ou alcényle comporte au moins 12 atomes de carbone. Comme sels, on utilise de préférence les bromures et chlorures tels que le chlorure de cocoyltriméthylammonium et le bromure de cétyltriméthylammonium.

Selon un mode de réalisation particulier de l'invention, on peut ajouter dans la dispersion aqueuse contenant les particules de gel cubique, un lipide amphiphile ionique non hydrosoluble, de préférence en une quantité allant de 0,0005 % à 5 % en poids et mieux de 0,001 % à 2 % en poids par rapport au poids total de la dispersion. Parmi les lipides amphiphiles ioniques non hydrosolubles, on peut notamment citer :
(i) les phospholipides tels que les phospholipides naturels comme la lécithine de soja ou d'oeuf, les phospholipides modifiés par voie chimique ou enzymatique comme la lécithine hydrogénée ou le sel de sodium de l'acide phosphatidique, et les phospholipides de synthèse comme la dipalmitoylphosphatidylcholine,
(ii) les esters phosphoriques d'acide gras et leurs sels, notamment de sodium et de potassium, tels que le monocétyl phosphate commercialisé sous la dénomination de "Monafax 160/" par la Société Mona, ainsi que le dimyristyl phosphate commercialisé sous la dénomination de « Mexoryl SY » par la Société Chimex,
(iii) les dérivés N-acylés de l'acide glutamique et leurs sels, tels que le stéaroylglutamate de monosodium commercialisé sous la dénomination de « Acylglutamate HS 11 » par la Société Ajinomoto, et le mélange cocoyl-alcoyl C14-C20 glutamate de monosodium, le radical alcoyle C14-C20 dérivant du suif hydrogéné, commercialisé sous la dénomination de « Acylglutamate GS 11 » par la Société Ajinomoto,
(iv) le cétylsulfate de sodium commercialisé sous la dénomination de « Nikkol SCS » par la Société Nikko Chemicals,
(v) le cocoyl monoglycéride sulfate de sodium commercialisé sous la dénomination de « Nikkol SGC 80 N » par la Société Nikko Chemicals, et
(vi) les dérivés d'ammonium quaternaire non hydrosolubles tels que le chlorure de béhényltriméthylammonium, le chlorure de dilauryldiméthylammonium, le chlorure de distéaryl-diméthylammonium, le méthylsulfate de 4,5-dihydro 1-méthyl 2-alcoyl C14-C20 1-(2-alcoyl C14-C20 aminoéthyl)imidazolium, les radicaux alcoyles C14-C20 dérivant du suif hydrogéné, commercialisé sous la dénomination de « Rewoquat W75H » par la Société Rewo Chemische, le méthylsulfate de dialkylhydroxyéthylméthylammonium dont les radicaux alkyles dérivent du suif, hydrogéné ou non, commercialisé sous la dénomination de « Stepanquat VP 85 » par la Société Stepan et le « Quaternium-82 » commercialisé par la Société Seppic sous la dénomination de « Amonyl DM ».

L'incorporation de ces lipides amphiphiles ioniques non hydrosolubles confère aux particules de gel cubique une charge superficielle entraînant une répulsion électrostatique des particules entre elles.

Les particules de gel cubique, telles que définies précédemment, ont une taille qui peut être modulée par la nature et la concentration des composés les constituant. Généralement, ces particules ont une taille moyenne en nombre, mesurée à l'aide d'un granulomètre laser BI 90 de la Société Brookhaven Instruments Corporation, d'environ 0,05 µm à environ 1 µm, et de préférence inférieure ou égale à 0,5 µm.

Il est en outre possible d'incorporer dans les particules de gel cubique, divers types de composés actifs. En particulier, lesdites particules peuvent contenir un principe actif hydrophile ou lipophile. Bien entendu, grâce à la structure particulière des particules de gel cubique, il est possible d'incorporer dans celles-ci à la fois des principes actifs hydrophiles et lipophiles, même si ceux-ci présentent une certaine incompatibilité.

Les compositions utilisées selon l'invention peuvent constituer notamment des compositions cosmétiques et dermatologiques. Elles contiennent pour une telle application, un milieu physiologiquement acceptable. On entend ici par « milieu physiologiquement acceptable », un milieu compatible avec la peau, et éventuellement avec les lèvres, le cuir chevelu, les cils, les yeux et/ou les cheveux. Ce milieu physiologiquement acceptable peut être plus particulièrement constitué d'eau et éventuellement d'un solvant organique physiologiquement acceptable choisi par exemple parmi les alcools inférieurs comportant de 1 à 4 atomes de carbone comme l'éthanol, l'isopropanol, le propanol, le butanol ; les polyéthylène glycols ayant de 6 à 80 motifs oxyde d'éthylène ; les polyols comme le propylène glycol, l'isoprène glycol, le butylène glycol, la glycérine, le sorbitol. Le milieu physiologiquement acceptable de la composition selon l'invention a un pH compatible avec la peau, et de préférence allant de 3 à 8 et mieux de 5 à 7.

Selon un mode préféré de réalisation, les compositions utilisées dans la présente invention comportent en outre une phase huileuse, apportant notamment du confort et de la douceur lors de l'application sur la peau. La quantité de phase huileuse peut aller par exemple de 2 à 40 % en poids et de préférence de 5 à 25 % en poids par rapport au poids total de la composition, le reste de la composition étant constitué de la phase aqueuse contenant le phytantriol ou constituée par les particules de gel cubique contenant le phytantriol ou contenant la dispersion aqueuse des particules de gel cubique contenant le phytantriol. Quand le phytantriol est dans des particules de gel cubique, le rapport en poids des composés amphiphiles constituant les particules de la phase cubique et de la phase huileuse va de préférence de 0,02/1 à 1/1, et mieux de 0,05/1 à 0,5/1.

Comme huiles utilisables dans l'invention, on peut citer les huiles minérales (huile de vaseline), les huiles végétales (fraction liquide du beurre de karité, huile de tournesol, huile d'amande d'abricot), les huiles animales (perhydrosqualène), les huiles de synthèse (polyisobutène hydrogénée, néopentanoate d'isostéaryle, myristate d'isopropyle), les huiles de silicone non volatiles ou volatiles (cyclométhicones telles que cyclopentasiloxane et cyclohexasiloxane) et les huiles fluorées (perfluoropolyéthers). On peut aussi utiliser, comme matières grasses, des alcools gras, des acides gras, des cires. La phase huileuse de l'émulsion peut contenir aussi des gommes telles que les gommes de silicone, des résines et des cires.

La composition contenant une phase huileuse peut être sous forme d'une émulsion eau-dans-huile (E/H) ou huile-dans-eau (H/E). Selon un mode préféré de réalisation, elle est sous forme d'une émulsion huile-dans-eau.

De façon connue, les compositions de l'invention peuvent contenir également des adjuvants habituels dans les domaines cosmétique ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur, les matières colorantes, les sels. Les quantités de ces différents adjuvants sont celles classiquement utilisées dans le domaine considéré, et par exemple de 0,01 à 20 % du poids total de la composition, et de préférence de 0,01 à 10 % du poids total de la composition. Ces adjuvants, selon leur nature, peuvent être introduits dans la phase grasse, dans la phase aqueuse et/ou dans des sphérules lipidiques.

Comme actifs, la composition peut contenir des actifs classiquement utilisés en cosmétique, tels que les agents empêchant l'adhésion des bactéries à la surface de la peau, les agents desquamants, les agents hydratants, les agents anti-séborrhéiques, les agents dépigmentants ou pro-pigmentants, les agents anti-glycation, les inhibiteurs de NO-synthase, les inhibiteurs de 5α-réductase, les inhibiteurs de lysyl et/ou prolyl hydroxylase, les agents stimulant la synthèse de macromolécules dermiques ou épidermiques et/ou empêchant leur dégradation, les agents stimulant la prolifération des fibroblastes ou des kératinocytes et/ou la différenciation des kératinocytes, les agents myorelaxants, les agents tenseurs, les agents antipollution ou anti-radicalaires, les agents apaisants, les actifs lipolytiques ou ayant une activité favorable, directe ou indirecte, sur la diminution du tissu adipeux, les agents agissant sur la microcirculation, et les agents agissant sur le métabolisme énergétique des cellules.

De façon avantageuse, outre le phytantriol, la composition peut contenir d'autres agents empêchant l'adhésion des bactéries à la surface de la peau tels que les huiles et les corps gras décrits dans la demande de brevet EP-1 313 086, ou les huiles végétales alcoxylées décrite dans la demande de brevet FR-2 832 057. Elle peut aussi avantageusement contenir d'autres actifs choisis parmi les agents desquamants, les agents hydratants, les agents anti-séborrhéiques et leurs mélanges.

Selon un autre mode de réalisation de l'invention, les compositions utilisées peuvent comporter en plus au moins un agent photoprotecteur organique et/ou au moins un agent photoprotecteur inorganique actif dans l'UVA et/ou l'UVB (absorbeurs), hydrosolubles ou liposolubles ou bien insolubles dans les solvants cosmétiques couramment utilisés.

Les agents photoprotecteurs organiques préférés sont choisis parmi l'éthylhexyl salicylate, l'éthylhexyl methoxycinnamate, l'octocrylene, le phenylbenzimidazole sulfonic acid, la benzophenone-3, la benzophenone-4, la benzophenone-5, la 4-methylbenzylidene camphor, le terephthalylidene dicamphor sulfonic acid, le disodium phenyl dibenzimidazole tetrasulfonate, la 2,4,6-tris-(4'-amino benzalmalonate de diisobutyle)-s-triazine, l'anisotriazine, l'éthylhexyl triazone, la diethylhexyl butamido triazone, le methylène bis-benzotriazolyl tetramethylbutylphénol, le drometrizole trisiloxane, et leurs mélanges.

Les agents photoprotecteurs inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les agents photoprotecteurs sont généralement présents dans les compositions selon l'invention dans des proportions allant de 0,1 à 20% en poids par rapport au poids total de la composition, et de préférence allant de 0,2 à 15% en poids par rapport au poids total de la composition.

Comme gélifiants, on peut citer par exemple les dérivés de cellulose tels que l'hydroxyéthylcellulose et les alkylhydroxyéthylcelluloses telles que la cétylhydroxyéthylcellulose ; les dérivés d'algues tels que le satiagum ; les gommes naturelles telles que l'adragante ou la gomme guar ; les polymères synthétiques tels que les polymères ou copolymères carboxyvinyliques et en particulier ceux commercialisés sous les dénominations de Carbopol^{R} par la société Goodrich ou de Synthalen^{R} par la société 3V SA. La proportion en agent gélifiant va de préférence de 0,1 à 2 % du poids total de la composition.

Les compositions utilisées selon l'invention peuvent être plus ou moins fluides et avoir l'aspect d'une crème blanche ou colorée, d'une pommade, d'un lait, d'une lotion, d'un sérum, d'une pâte, d'une mousse. Elles peuvent être éventuellement appliquées sur la peau sous forme d'aérosol. Elles peuvent aussi se présenter sous forme solide, et par exemple sous forme de stick.

De préférence, les compositions utilisées selon l'invention sont obtenues selon un procédé de préparation comprenant au moins deux étapes. La première étape consiste généralement à préparer une dispersion aqueuse de particules de gel cubique, telles que définies précédemment, par fragmentation, à l'aide d'un homogénéiseur, des composés tels que définis précédemment et d'eau, en présence éventuellement de lipides amphiphiles ioniques non hydrosolubles et/ou de principes actifs hydrophiles et/ou lipophiles et/ou d'un agent dispersant et stabilisant tels que définis précédemment. L'homogénéiseur peut être du type rotor-stator à fort gradient de cisaillement comme le Virtis^{R} ou le Heidolph Diax 600^{R} ou un homogénéiseur haute pression fonctionnant entre 200 et 1.800 bars environ (20 à 180 MPa).

Il est bien entendu possible d'introduire, à ce stade de la préparation de la dispersion aqueuse des particules de gel cubique, divers additifs et/ou principes actifs dans la phase aqueuse. Après la formation des particules de gel cubique, l'agent dispersant et stabilisant se trouve, généralement, à l'extérieur desdites particules.

La deuxième étape consiste généralement ensuite à ajouter à ladite dispersion obtenue une phase huileuse contenant éventuellement certains additifs et/ou principes actifs lipophiles et à soumettre le mélange à une agitation mécanique qui peut être notamment réalisée à l'aide d'un homogénéiseur du même type que ceux définis ci-dessus.

Divers additifs et/ou principes actifs peuvent être également introduits à ce stade de la préparation. Par ailleurs, lorsque l'on souhaite préparer une dispersion gélifiée, dans une troisième étape, on ajoute généralement au mélange obtenu après la seconde étape, une solution aqueuse contenant un agent gélifiant.

L'utilisation du phytantriol selon l'invention trouve une application plus spécifiquement dans le domaine cosmétique ou dermatologique. En particulier, les compositions contenant le phytantriol pourront être utilisées pour le nettoyage et/ou le démaquillage et/ou le soin de la peau. Le phytantriol peut aussi être utilisé dans le cadre de la présente invention dans des produits solaires, dans des produits de maquillage comme les fonds de teint, dans les rouges à lèvres, les mascaras, les fards, et/ou dans les déodorants.

La présente invention concerne également un procédé cosmétique pour prévenir et/ou lutter contre les désordres liés à l'adhésion des micoorganismes comprenant l'application sur la peau et/ou les muqueuses d'une composition selon l'invention, contenant du phytantriol dans un milieu physiologiquement acceptable.

Plus spécifiquement l'invention concerne un procédé de traitement cosmétique non thérapeutique des peaux grasses comprenant l'application topique d'une composition selon l'invention, contenant du phytantriol dans un milieu physiologiquement acceptable.

Un aspect particulier de l'invention concerne l'utilisation non thérapeutique de phytantriol, en tant qu'agent empêchant ou réduisant l'adhésion des microorganismes sur la surface de la peau et/ou des muqueuses dans une composition démaquillante de la peau.

Ainsi un autre objet de la présente invention concerne un procédé cosmétique non thérapeutique de nettoyage et/ou de démaquillage de la peau comprenant l'application topique d'une composition contenant du phytantriol selon l'invention, dans un milieu physiologiquement acceptable.

La flore microbienne de la surface de la peau étant responsable d'un grand nombre de désordres allant du simple désagrément (odeur, boutons) aux maladies plus graves, la présente invention a aussi pour objet l'utilisation cosmétique de phytantriol, en tant qu'agent destiné à diminuer les mauvaises odeurs corporelles, et/ou destiné aux soins d'hygiène corporelle.

Un autre aspect de l'invention concerne donc un procédé cosmétique non thérapeutique pour réduire les mauvaises odeurs corporelles comprenant l'application topique d'une composition selon l'invention contenant du phytantriol dans un milieu physiologiquement acceptable.

La présente invention a également pour objet l'utilisation de phytantriol pour la préparation d'une composition dermatologique destinée à prévenir et/ou lutter contre l'acné, particulièrement l'acné juvénile. En particulier, la composition ne contient pas d'autre agent anti-acné que le phytantriol.

La présente invention a aussi pour objet l'utilisation de phytantriol pour la préparation d'une composition dermatologique destinée à prévenir et/ou lutter contre les mycoses. En particulier, la composition ne contient pas d'autre agent anti-mycotique que le phytantriol.

Par "*destiné aux soins d'hygiène corporelle*" on entend une substance destinée à être mise en contact avec les diverses parties superficielles du corps humain et/ou avec la muqueuses, et/ou avec les dents, en vue de les nettoyer, de les protéger, de les maintenir en bon état, d'en modifier l'aspect, de les parfumer, ou d'en corriger l'odeur.

Les exemples qui suivent servent à illustrer l'invention sans toutefois présenter un caractère limitatif. Les composés sont, selon le cas, cités en noms chimiques ou en noms CTFA (International Cosmetic Ingredient Dictionary and Handbook).

### Exemple 1 :

| | | |
|---|---|---|
| Phase A | Phytantriol | 2,94 % |
| | Phosphate de mono-cétyle | 0,06 % |
| | Eau | 1,6 % |
| Phase B | Polysorbate 40 | 1 % |
| | Glycérine | 4 % |
| | Eau | 46,15 % |
| Phase C | Graisse de sal | 2 % |
| | Octyldodécanol | 2 % |
| | Huile d'abricot | 6 % |
| | Stéarate d'isocétyle | 3 % |
| | Cyclohexasiloxane | 6 % |
| | Filtre UV | 1 % |
| | Parfum | 0,2 % |
| Phase D | Hydroxyéthylcéllulose | 1 % |
| | Pentasodium éthylènediamine tétraméthylène | |
| | Phosphonate | 0,033 % |
| | Eau | 23,017 % |

### Mode opératoire :

Le mélange des phases A et B est placé sous agitation à 60-70°C jusqu'à homogénéisation, puis refroidi à 25°C.
La phase C est ensuite chauffée vers 50°C et homogénéisée à l'aide d'un agitateur magnétique, puis refroidie à température ambiante.
La phase C est ensuite ajoutée au mélange A et B à température ambiante sous agitation pendant 15 mn.
Le mélange obtenu est ensuite passé deux fois à l'homogénéisateur à Haute Pression. (600 bars à température ambiante).
La préparation obtenue est alors gélifiée à l'aide du mélange de la phase D (préalablement gonflée sous Rayneri à température ambiante.

On obtient alors une crème homogène qui, après application sur la peau, empêche l'adhésion des bactéries à la surface de la peau. Cette crème est appropriée pour le traitement des mycoses et/ou de l'acné.

### Exemple 2 : Test d'anti-adhésion microbienne :

### Protocole :

L'activité du phytantriol a été mise en évidence sur épiderme reconstruit.
Avant l'adhésion bactérienne, l'épiderme reconstruit est mis en contact pendant 2 heures avec la composition de l'exemple 1 à 37°C. On y ajoute alors 1 ml de suspension bactérienne de *Staphylococcus aureus* à une concentration de 10⁷ germes/ml dans du Tryptone sel. Après incubation 24 heures à 37°C, la suspension bactérienne est vidée et cinq rinçages sont réalisés par 1 ml d'eau distillée stérile. L'épiderme reconstruit détaché de son support est alors broyé à l'aide d'un robot dans 18 ml de Tryptone sel. On effectue une dilution décimale de cette suspension dans le Tryptone sel, on procède ensuite à un ensemencement de 1 ml de la dilution dans 15 ml de la gélose Trypticase Soja et à l'incubation pendant 24 heures à 37°C. On dénombre ensuite les cellules adhérentes et viables.

Ce test d'anti-adhésion permet d'évaluer l'efficacité de molécules seules ou de produits finis.

Avant le test d'anti-adhésion, on met en oeuvre le test de viabilité suivant :

Un mélange bactéries/produit à tester, dans le même rapport que dans le test anti-adhésion, est mis en contact 24 heures à 37°C. Le dénombrement des germes est réalisé par dilution décimale dans du Tryptone sel et ensemencement au râteau de 100 µl sur de la gélose Trypticase Soja. Le comptage des colonies s'effectue après 24 heures d'incubation à 37°C.

L'essai de viabilité réalisé préalablement au test d'anti-adhésion permet d'écarter toute composante bactéricide des molécules ou des produits finis testés et de ne mettre en évidence que l'activité anti-adhésion.

### Résultats :

Les résultats obtenus sont récapitulés dans le tableau suivant :

| **Formule testée** | **Activité** | **anti-adhésion** |
|---|---|---|
| | Résultats quantitatifs | Résultats qualitatifs |
| Composition de l'exemple 1 | -1,7 | Bonne |
| Phytantriol pur | -1,9 | Bonne |

Les résultats quantitatifs correspondent à la diminution du logarithme décimal du nombre moyen de *Staphylococcus aureus* viable adhérent sur l'épiderme reconstruit après traitement par la composition ou la molécules testées par rapport au logarithme décimal du nombre moyen de *Staphylococcus aureus* viable adhérent sur l'épiderme reconstruit après traitement à l'eau dans les mêmes conditions. Les résultats sont considérés comme significatifs si la différence est supérieure à 0,5 log.

Les résultats qualitatifs obtenus pour la composition et pour le phytantriol pur sont exprimés par un terme variant en fonction des valeurs de Log de réduction de l'adhésion du germes au bout de 24 heures par rapport au même test utilisant l'eau :
- résultats supérieurs à ceux obtenus avec l'eau Pro-adhésion.
- résultats identiques à ceux obtenus avec l'eau Sans effet,
- entre 0.5 et 1 log de réduction par rapport à l'eau Faible,
- entre 1 et 1.5 log de réduction par rapport à l'eau Moyenne,
- 1.5 et 2 log de réduction par rapport à l'eau Bonne,
- 2 Log et plus de réduction par rapport à l'eau Excellente,

## Revendications

1. Utilisation cosmétique du phytantriol dans une composition en tant qu'agent empêchant ou réduisant l'adhésion des microorganismes sur la surface de la peau et/ou des muqueuses.

2. Utilisation du phytantriol pour la préparation d'une composition dermatologique destinée à prévenir ou lutter contre les pathologies liées à l'adhésion des microorganismes sur la surface de la peau et/ou des muqueuses.

3. Utilisation selon l'une des revendications 1 ou 2 **caractérisée en ce que** le phytantriol est présent dans la composition pour application topique, en une quantité allant de 0,001 à 20 % en poids et de préférence de 0,1 à 10 % en poids par rapport au poids total de la composition.

4. Utilisation selon l'une des revendications précédentes **caractérisées en ce que** le phytantriol est sous forme de particules de gel cubique.

5. Utilisation selon la revendication 4 **caractérisée en ce que** les particules de gel cubique contenant le phytantriol sont en dispersion aqueuse.

6. Utilisation selon l'une des revendications 1 à 5 **caractérisée en ce que** les particules de gel cubique sont formées par un mélange comprenant (i) de 0,1 à 15 % en poids par rapport au poids total de la composition de 3,7,11,15-tétraméthyl-1,2,3-hexadecanetriol ou phytantriol, éventuellement associé à un dérivé de N-2-alcoxycarbonyle de N-méthylglucamine et/ou un monoglycéride d'acide gras insaturé, et (ii) de 0,05 à 3 % en poids par rapport au poids total de la composition, d'au moins un agent dispersant et stabilisant, ledit agent étant choisi parmi les agents tensioactifs hydrosolubles à température ambiante, contenant une chaîne grasse, saturée ou insaturée, linéaire ou ramifiée, ayant de 8 à 22 atomes de carbone.

7. Utilisation selon la revendication 6 **caractérisée en ce que** la proportion pondérale relative en composé (i) par rapport au poids à l'agent dispersant et stabilisant (ii) est comprise entre 2 à 200.

8. Utilisation selon l'une des revendications 6 ou 7 **caractérisée par le fait que** le dérivé N-2-alcoxycarbonyle de N-méthylglucamine répond à la formule (I) suivante : 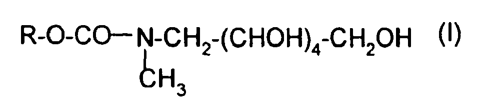 dans laquelle R représente un radical alkyle ramifié comportant de 6 à 18 atomes de carbone.

9. Utilisation selon l'une des revendications 6 à 8 **caractérisée en ce que** le dérivé N-2-alcoxycarbonyles de N-méthylglucamine est choisi parmi la N-2-hexyldécyloxycarbonyl-N-méthylglucamine, la N-2-éthylhexyloxycarbonyl-N-méthylglucamine, la N-2-butyloctyloxycarbonyl-N-méthylglucamine et leurs mélanges.

10. Utilisation selon l'une quelconque des revendications 6 à 9, **caractérisée par le fait que** les particules de gel cubique contiennent comme composé (i), un mélange constitué de 1 à 40 % en poids de phytantriol par rapport au poids du mélange et de 60 à 99 % en poids de dérivé N-2-alcoxycarbonyle de N-méthylglucamine par rapport au poids du mélange.

11. Utilisation selon la revendication 6, **caractérisée par le fait que** ledit monoglycéride d'acide gras insaturé est choisi parmi le monooléate de glycéryle et le monolinoléate de glycéryle.

12. Utilisation selon la revendication 6, **caractérisée par le fait que** les particules de gel cubique contiennent comme composé (i) un mélange constitué de 1 à 50 % en poids de phytantriol par rapport au poids du mélange, et de 50 à 99 % en poids de monoglycéride d'acide gras insaturé par rapport au poids du mélange.

13. Utilisation selon l'une quelconque des revendications 6 à 12, **caractérisée par le fait que** ledit agent dispersant et stabilisant est choisi parmi :
(1) les alkyl ou alcényl éthers ou esters de polyol,
(2) les amino-acides N-acylés et leurs dérivés et les peptides N-acylés par un radical alkyle ou alcényle, et leurs sels,
(3) les alkyl ou alcényl éthers ou esters sulfates, leurs dérivés et leurs sels,
(4) les alkyl ou alcényl éthers ou esters gras polyoxyéthylénés,
(5) les acides alkyl ou alcényl carboxyliques polyoxyéthylénés et leurs sels,
(6) les N-alkyl ou alcényl bétaïnes,
(7) les alkyl ou alcényl triméthylammonium et leurs sels, et
(8) leurs mélanges.

14. Utilisation selon l'une quelconque des revendications 4 à 13, **caractérisée par le fait que** les particules de gel cubique ont une taille allant de 0,05 µm à 1 µm.

15. Utilisation selon l'une quelconque des revendications 5 à 14, **caractérisée par le fait que** la dispersion de particules de gel cubique comprend en outre au moins un lipide amphiphile ionique non hydrosoluble.

16. Utilisation selon la revendication 15, **caractérisée par le fait que** ledit lipide amphiphile ionique non hydrosoluble est choisi parmi :
(i) les phospholipides,
(ii) les esters phosphoriques d'acide gras,
(iii) les dérivés N-acylés de l'acide glutamique non hydrosolubles et leurs sels,
(iv) le cétylsulfate de sodium,
(v) le cocoyl monoglycéride sulfate de sodium, et
(vi) les dérivés d'ammonium quaternaire non hydrosolubles.

17. Utilisation selon l'une quelconque des revendications 4 à 16, **caractérisée par le fait que** les particules comprennent au moins un principe actif hydrophile et/ou lipophile.

18. Utilisation selon l'une quelconque des revendications 4 à 16, **caractérisée par le fait que** les particules de gel cubique sont présentes en une quantité allant de 0,1 à 20 % en poids par rapport au poids total de la composition.

19. Utilisation selon l'une des revendications 1 à 18 **caractérisée en ce que** les compositions utilisées comportent en plus au moins un agent photoprotecteur organique et/ou au moins un agent photoprotecteur inorganique actif dans l'UVA et/ou l'UVB (absorbeurs), hydrosolubles ou liposolubles ou bien insolubles dans les solvants cosmétiques couramment utilisés.

20. Procédé cosmétique non thérapeutique de nettoyage et/ou de démaquillage de la peau comprenant l'application topique d'une composition telle que définie dans l'une des revendications 1 à 19, contenant du phytantriol dans un milieu physiologiquement acceptable.

21. Procédé cosmétique non thérapeutique pour prévenir et/ou lutter contre les désordres liés à l'adhésion des microorganismes comprenant l'application sur la peau et/ou les muqueuses d'une composition telle que définie dans l'une des revendications 1 à 19, contenant du phytantriol dans un milieu physiologiquement acceptable.

22. Procédé de traitement cosmétique non thérapeutique des peaux grasses comprenant l'application topique d'une composition telle que définie dans l'une des revendications 1 à 19, contenant du phytantriol dans un milieu physiologiquement acceptable.

23. Procédé cosmétique non thérapeutique pour réduire les mauvaises odeurs corporelles comprenant l'application topique d'une composition telle que définie dans l'une des revendications 1 à 19, contenant du phytantriol dans un milieu physiologiquement acceptable.

24. Utilisation de phytantriol pour la préparation d'une composition dermatologique destinée à prévenir et/ou lutter contre l'acné, particulièrement l'acné juvénile.

25. Utilisation selon la revendication précédente, **caractérisée par le fait que** la composition ne contient pas d'autre agent anti-acné que le phytantriol.

26. Utilisation de phytantriol pour la préparation d'une composition dermatologique destinée à prévenir et/ou lutter contre les mycoses.

27. Utilisation selon la revendication précédente, **caractérisée par le fait que** la composition ne contient pas d'autre agent anti-mycotique que le phytantriol.

## Claims

1. Cosmetic use of phytantriol in a composition as an agent for preventing or reducing the adhesion of microorganisms to the surface of the skin and/or mucous membranes.

2. Use of phytantriol for the preparation of a dermatological composition for use in preventing or combating pathologies associated with the adhesion of microorganisms to the surface of the skin and/or mucous membranes.

3. Use according to either of Claims 1 and 2, **characterized in that** the phytantriol is present in the composition for topical application in an amount ranging from 0.001% to 20% by weight and preferably from 0.1% to 10% by weight, relative to the total weight of the composition.

4. Use according to one of the preceding claims, **characterized in that** the phytantriol is in the form of cubic gel particles.

5. Use according to Claim 4, **characterized in that** the cubic gel particles containing the phytantriol are in aqueous dispersion.

6. Use according to one of Claims 1 to 5, **characterized in that** the cubic gel particles are formed from a mixture comprising (i) from 0.1% to 15% by weight, relative to the total weight of the composition, of 3,7,11,15-tetramethyl-1,2,3-hexadecanetriol or phytantriol, optionally combined with an N-2-alkoxycarbonyl N-methylglucamine derivative and/or an unsaturated fatty acid monoglyceride, and (ii) from 0.05% to 3% by weight, relative to the total weight of the composition, of at least one dispersing and stabilizing agent, said agent being chosen from surfactants that are water-soluble at ambient temperature, containing a saturated or unsaturated, linear or branched fatty chain containing from 8 to 22 carbon atoms.

7. Use according to Claim 6, **characterized in that** the relative weight proportion of compound (i) relative to the weight of the dispersing and stabilizing agent (ii) is between 2 and 200.

8. Use according to either of Claims 6 and 7, **characterized in that** the N-2-alkoxycarbonyl N-methylglucamine derivative corresponds to formula (I) below: 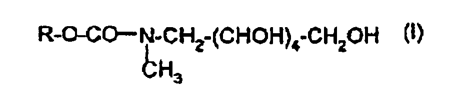 in which R represents a branched alkyl radical containing from 6 to 18 carbon atoms.

9. Use according to one of Claims 6 to 8, **characterized in that** the N-2-alkoxycarbonyl N-methylglucamine derivative is chosen from N-2-hexyldecyloxycarbonyl-N-methylglucamine, N-2-ethylhexyloxycarbonyl-N-methylglucamine and N-2-butyloctyloxycarbonyl-N-methylglucamine, and mixtures thereof.

10. Use according to any one of Claims 6 to 9, **characterized in that** the cubic gel particles contain, as compound (i), a mixture consisting of from 1 to 40% by weight of phytantriol, relative to the weight of the mixture, and from 60% to 99% by weight of N-2-alkoxycarbonyl N-methylglucamine derivative, relative to the weight of the mixture.

11. Use according to Claim 6, **characterized in that** said unsaturated fatty acid monoglyceride is chosen from glyceryl monooleate and glyceryl monolinoleate.

12. Use according to Claim 6, **characterized in that** the cubic gel particles contain, as compound (i), a mixture consisting of from 1% to 50% by weight of phytantriol, relative to the weight of the mixture, and from 50% to 99% by weight of unsaturated fatty acid monoglyceride, relative to the weight of the mixture.

13. Use according to any one of Claims 6 to 12, **characterized in that** said dispersing and stabilizing agent is chosen from:
(1) alkyl or alkenyl ethers or esters of a polyol,
(2) N-acyl amino acids and derivatives thereof, and peptides N-acylated with an alkyl or alkenyl radical, and salts thereof,
(3) alkyl or alkenyl ether or ester sulphates, derivatives thereof and salts thereof,
(4) polyoxyethylenated fatty alkyl or alkenyl ethers or esters,
(5) polyoxyethylenated alkyl or alkenyl carboxylic acids and salts thereof,
(6) N-alkyl or alkenyl betaines,
(7) alkyl or alkenyl trimethylammonium and salts thereof, and
(8) mixtures thereof.

14. Use according to any one of Claims 4 to 13, **characterized in that** the cubic gel particles having a size ranging from 0.05 µm to 1 µm.

15. Use according to any one of Claims 5 to 14, **characterized in that** the dispersion of cubic gel particles also comprises at least one water-insoluble ionic amphiphilic lipid.

16. Use according to Claim 15, **characterized in that** said water-insoluble ionic amphiphilic lipid is chosen from:
(i) phospholipids,
(ii) phosphoric esters of fatty acids,
(iii) water-insoluble N-acyl derivatives of glutamic acid and salts thereof,
(iv) sodium cetyl sulphate,
(v) sodium cocoyl monoglyceride sulphate, and
(vi) water-insoluble quaternary ammonium derivatives.

17. Use according to any one of Claims 4 to 16, **characterized in that** the particles comprise at least one hydrophilic and/or lipophilic active principle.

18. Use according to any one of Claims 4 to 16, **characterized in that** the cubic gel particles are present in an amount ranging from 0.1% to 20% by weight relative to the total weight of the composition.

19. Use according to one of Claims 1 to 18, **characterized in that** the compositions used also comprise at least one organic photoprotective agent and/or at least one inorganic photoprotective agent active in the UVA and/or UVB range (absorbers), which are water-soluble or liposoluble or even insoluble in the cosmetic solvents commonly used.

20. Non-therapeutic cosmetic process for cleansing and/or removing makeup from the skin, comprising the topical application of a composition as defined in one of Claims 1 to 19, containing phytantriol in a physiologically acceptable medium.

21. Non-therapeutic cosmetic process for preventing and/or combating disorders associated with the adhesion of microorganisms, comprising the application to the skin and/or mucous membranes of a composition as defined in one of Claims 1 to 19, containing phytantriol in a physiologically acceptable medium.

22. Non-therapeutic cosmetic process for treating greasy skin, comprising the topical application of a composition as defined in one of Claims 1 to 19, containing phytantriol in a physiologically acceptable medium.

23. Non-therapeutic cosmetic process for reducing unpleasant body odour, comprising the topical application of a composition as defined in one of Claims 1 to 19, containing phytantriol in a physiologically acceptable medium.

24. Use of phytantriol for the preparation of a dermatological composition for use in preventing and/or combating acne, particularly juvenile acne.

25. Use according to the preceding claim, **characterized in that** the composition contains no antiacne agent other than phytantriol.

26. Use of phytantriol for the preparation of a dermatological composition for use in preventing and/or combating mycoses.

27. Use according to the preceding claim, **characterized in that** the composition contains no antimycotic agent other than phytantriol.

## Patentansprüche

1. Kosmetische Verwendung von Phytantriol in einer Zusammensetzung als Mittel zur Verhinderung oder Verminderung des Anhaftens von Mikroorganismen an der Oberfläche der Haut und/oder der Schleimhäute.

2. Verwendung von Phytantriol zur Herstellung einer dermatologischen Zusammensetzung zur Vorbeugung oder Bekämpfung pathologischer Zustände, die mit dem Anhaften von Mikroorganismen an der Oberfläche der Haut und/oder der Schleimhäute verbunden sind.

3. Verwendung nach Anspruch 1 oder 2, **dadurch gekennzeichnet, dass** das Phytantriol in der Zusammensetzung zur topischen Anwendung in einer Menge von 0,001 bis 20 Gew.-% und vorzugsweise von 0,1 bis 10 Gew.-% vorliegt, bezogen auf das Gesamtgewicht der Zusammensetzung.

4. Verwendung nach einem der vorhergehenden Ansprüche, **dadurch gekennzeichnet, dass** das Phytantriol in Form von Partikeln eines kubischen Gels vorliegt.

5. Verwendung nach Anspruch 4, **dadurch gekennzeichnet, dass** die Partikel des kubischen Gels, welches das Phytantriol enthält, in wässeriger Dispersion vorliegen.

6. Verwendung nach einem der Ansprüche 1 bis 5, **dadurch gekennzeichnet, dass** die Partikel des kubischen Gels aus einem Gemisch hergestellt sind, das enthält: (i) 0,1 bis 15 Gew.-%, bezogen auf das Gesamtgewicht der Zusammensetzung, 3,7,11,15-Tetramethyl-1,2,3-hexadecantriol oder Phytantriol, gegebenenfalls in Kombination mit einem N-2-Alkoxycarbonyl-Derivat von N-Methylglucamin und/oder einem Monoglycerid einer ungesättigten Fettsäure, und (ii) 0,05 bis 3 Gew.-%, bezogen auf das Gesamtgewicht der Zusammensetzung, mindestens eines Dispergier- und Stabilisierungsmittels, das unter bei Umgebungstemperatur wasserlöslichen grenzflächenaktiven Mitteln ausgewählt ist, die eine gesättigte oder ungesättigte, geradkettige oder verzweigte Fettkette mit 8 bis 22 Kohlenstoffatomen enthalten.

7. Verwendung nach Anspruch 6, **dadurch gekennzeichnet, dass** das relative Gewichtsverhältnis des Gewichts der Verbindung (i) zum Gewicht des Dispergier- und Stabilisierungsmittels (ii) im Bereich von 2 bis 200 liegt.

8. Verwendung nach Anspruch 6 oder 7, **dadurch gekennzeichnet, dass** das N-2-Alkoxycarbonyl-Derivat von N-Methylglucamin der nachstehenden Formel (I) entspricht, 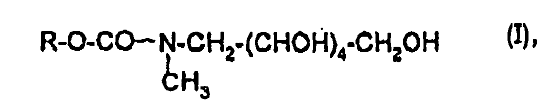 in der R eine verzweigte Alkylgruppe mit 6 bis 18 Kohlenstoffatomen bedeutet.

9. Verwendung nach einem der Ansprüche 6 bis 8, **dadurch gekennzeichnet, dass** das N-2-Alkoxycarbonyl-Derivat von N-Methylglucamin ausgewählt ist unter N-2-Hexyldecyloxycarbonyl-N-methylglucamin, N-2-Ethylhexyloxycarbonyl-N-methylglucamin und N-2-Butyloctyloxycarbonyl-N-methylglucamin sowie Gemischen dieser Verbindungen.

10. Verwendung nach einem der Ansprüche 6 bis 9, **dadurch gekennzeichnet, dass** die Partikel des kubischen Gels als Verbindung (i) ein Gemisch enthalten, das besteht aus 1 bis 40 Gew.% Phytantriol, bezogen auf das Gewicht des Gemisches, und 60 bis 99 Gew.-% N-2-Alkoxycarbonyl-Derivat von N-Methylglucamin, bezogen auf das Gewicht des Gemisches.

11. Verwendung nach Anspruch 6, **dadurch gekennzeichnet, dass** das Monoglycerid der ungesättigten Fettsäure unter Glycerylmonooleat und Glycerylmonolinoleat ausgewählt ist.

12. Verwendung nach Anspruch 6, **dadurch gekennzeichnet, dass** die Partikel des kubischen Gels als Verbindung (i) ein Gemisch enthalten, das besteht aus 1 bis 50 Gew.-% Phytantriol, bezogen auf das Gewicht des Gemisches, und 50 bis 99 Gew.-% Monoglycerid der ungesättigten Fettsäure, bezogen auf das Gewicht des Gemisches.

13. Verwendung nach einem der Ansprüche 6 bis 12, **dadurch gekennzeichnet, dass** das Diapergier- und Stabilisierungsmittel ausgewählt ist unter
(1) Alkyl- oder Alkenylethern oder Estern von Polyolen,
(2) N-acylierten Aminosäuren und ihren Derivaten und N-acylierten Peptiden, die mit einer Alkylgruppe oder Alkenylgruppe acyliert sind, sowie ihren Salzen,
(3) Alkyl- oder Alkenylethersulfaten oder Alkyl- oder Alkenylestersulfaten, ihren Derivaten und ihren Salzen,
(4) Alkyl- oder Alkenylethern mit Fettkette oder Alkyl- oder Alkenylestern mit Fettkette, die polyoxyethyleniert sind,
(5) polyoxyethylenierten Alkyl- oder Alkenylcarbonsäuren und ihren Salzen,
(6) N-Alkyl- oder -alkenylbetainen,
(7) Alkyl- oder Alkenyltrimethylammoniumverbindungen und ihren Salzen sowie
(8) den Gemischen dieser Verbindungen.

14. Verwendung nach einem der Ansprüche 4 bis 13, **dadurch gekennzeichnet, dass** die Partikel des kubischen Gels eine Größe von 0,05 bis 1 µm aufweisen.

15. Verwendung nach einem der Ansprüche 5 bis 14, **dadurch gekennzeichnet, dass** die Dispersion der Partikel des kubischen Gels ferner mindestens ein nicht wasserlösliches, ionisches amphiphiles Lipid enthält.

16. Verwendung nach Anspruch 15, **dadurch gekennzeichnet, dass** das nicht wasserlösliche ionische amphiphile Lipid ausgewählt ist unter
(i) Phospholipiden,
(ii) Phosphorsäureestern von Fettsäuren,
(iii) nicht wasserlöslichen N-Acylderivaten von Glutaminsäure und ihren Salzen,
(iv) Natriumcetylsulfat,
(v) Natriumcocoylmonoglyceridsulfat und
(vi) nicht wasserlöslichen quaternären Ammoniumderivaten.

17. Verwendung nach einem der Ansprüche 4 bis 16, **dadurch gekennzeichnet, dass** die Partikel mindestens einen hydrophilen und/oder lipophilen Wirkstoff enthalten.

18. Verwendung nach einem der Ansprüche 4 bis 16, **dadurch gekennzeichnet, dass** die Partikel des kubischen Gels in einer Menge von 0,1 bis 20 Gew.-% vorliegen, bezogen auf das Gesamtgewicht der Zusammensetzung.

19. Verwendung nach einem der Ansprüche 1 bis 18, **dadurch gekennzeichnet, dass** die verwendeten Zusammensetzungen ferner mindestens ein organisches Lichtschutzmittel und/oder mindestens ein anorganisches Lichtschutzmittel, das im UV-A und/oder im UV-B wirksam ist (Absorber), enthält, wobei die Lichtschutzmittel wasserlöslich oder öllöslich oder in üblicherweise verwendeten kosmetischen Lösungsmitteln unlöslich sind.

20. Nichttherapeutisches kosmetisches Verfahren zur Reinigung und/oder zum Abschminken der Haut, das die topische Anwendung einer Zusammensetzung umfasst, wie sie in einem der Ansprüche 1 bis 19 definiert ist und die Phytantriol in einem physiologisch akzeptablen Medium enthält.

21. Nichttherapeutisches kosmetisches Verfahren zur Vorbeugung und/oder zur Bekämpfung von mit dem Anhaften von Mikroorganismen in Verbindung stehenden Störungen, das die Anwendung einer Zusammensetzung, wie sie in einem der Ansprüche 1 bis 19 definiert ist, und die Phytantriol in einem physiologisch akzeptablen Medium enthält, auf die Haut und/oder die Schleimhäute umfasst.

22. Verfahren zur rrichttherapeutischen kosmetischen Behandlung von fetter Haut, das die topische Anwendung einer Zusammensetzung umfasst, wie sie in einem der Ansprüche 1 bis 19 definiert ist und die Phytantriol in einem physiologisch akzeptablen Medium enthält.

23. Nichttherapeutisches kosmetisches Verfahren zur Verminderung von schlechtem Körpergeruch, das die topische Anwendung einer Zusammensetzung umfasst, wie sie in einem der Ansprüche 1 bis 19 definiert ist und die Phytantriol in einem physiologisch akzeptablen Medium enthält.

24. Verwendung von Phytantriol zur Herstellung einer dermatologischen Zusammensetzung zur Vorbeugung und/oder zur Bekämpfung von Akne, insbesondere von juveniler Akne.

25. Verwendung nach dem vorhergehenden Anspruch, **dadurch gekennzeichnet, dass** die Zusammensetzung kein anderes Antiaknemittel als Phytantriol enthält.

26. Verwendung von Phytantriol zur Herstellung einer dermatologischen Zusammensetzung zur Vorbeugung und/oder zur Bekämpfung von Mykosen.

27. Verwendung nach dem vorhergehenden Anspruch, **dadurch gekennzeichnet, dass** die Zusammensetzung kein anderes antimykotisches Mittel als Phytantriol enthält.
